# EUROPÄISCHE PATENTSCHRIFT

(11) **EP 3 229 761 B1**
(45) Veröffentlichungstag und Bekanntmachung des Hinweises auf die Patenterteilung: **12.08.2020**
(21) Anmeldenummer: 15787622.8
(22) Anmeldetag: 02.11.2015
(51) Int. Cl.: A61K 8/34, A61Q 5/06, A61K 8/04, A61K 8/20

(54) **MITTEL UND VERFAHREN ZUR TEMPORÄREN VERFORMUNG KERATINHALTIGER FASERN**
AGENTS AND METHODS FOR THE TEMPORARY SHAPING OF KERATIN-CONTAINING FIBERS
PRODUIT ET PROCÉDÉS DE MISE EN EN FORME TEMPORAIRE DE FIBRES KÉRATINIQUES

(30) Priorität: 10.12.2014 DE 102014225420
(43) Veröffentlichungstag der Anmeldung: 18.10.2017
(73) Patentinhaber: Henkel AG & Co. KGaA, 40589 Düsseldorf (DE)
(72) Erfinder: KNAPPE, Thorsten, 22869 Schenefeld (DE); BAYERSDÖRFER, Rolf, 22589 Hamburg (DE); FÖRSTER, Thomas, 40591 Düsseldorf (DE)
(86) Internationale Anmeldenummer: PCT/EP2015/075376
(87) Internationale Veröffentlichungsnummer: WO 2016/091463

(56) Entgegenhaltungen:
- WO-A1-01/70179
- WO-A1-99/15135
- DE-A1- 2 525 667
- DE-A1- 2 608 649
- JP-A- 2007 319 234
- JP-A- 2009 136 571
- US-A- 3 372 840
- US-A1- 2002 031 478
- US-A1- 2002 074 349
- US-A1- 2002 079 377
- US-A1- 2004 065 683
- US-A1- 2008 152 610
- US-A1- 2012 301 419
- US-A1- 2013 018 333
- US-A1- 2013 164 246

## Beschreibung

Die Anmeldung betrifft das technische Fachgebiet der temporären Umformung keratinhaltiger Fasern, insbesondere menschlicher Haare. Gegenstand der Anmeldung sind spezifische haarkosmetische Formulierungen, welche sich zur Applikation auf keratinhaltige Fasern mittels eines Entspannungsverdampfungsverfahrens eignen. Darüber hinaus sind die Verwendung dieser haarkosmetischen Formulierungen in Vorrichtungen zur Entspannungsverdampfung und Verfahren zur temporären Umformung keratinhaltiger Fasern Gegenstand der vorliegenden Anmeldung

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, welche sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, welche einer permanenten oder temporären Formgebung der Haare dienen, eine wichtige Rolle. Während bei der permanenten Umformung die chemische Struktur der keratinhaltigen Faser durch Reduktion und Oxidation modifiziert wird, finden solche Modifikationen der chemischen Struktur bei der temporären Umformung nicht statt. Entsprechende Mittel zur temporären Verformung enthalten als festigenden Wirkstoff üblicherweise synthetische Polymere und/oder Wachse.

Die wichtigste Eigenschaft eines Mittels zur temporären Verformung keratinischer Fasern, im Folgenden auch Stylingmittel genannt, besteht darin, den behandelten Fasern in der neu modellierten Form - d.h. einer den Fasern aufgeprägten Form - einen möglichst starken Halt zu geben. Handelt es sich bei den keratinischen Fasern um menschliche Haare, spricht man auch von starkem Frisurenhalt oder von hohen Haltegrad des Stylingmittels. Der Frisurenhalt wird im Wesentlichen durch die Art und Menge der eingesetzten festigenden Wirkstoffe bestimmt, wobei jedoch auch ein Einfluss der weiteren Bestandteile des Stylingmittels sowie der Applikationsform gegeben sein kann.

Im Bereich der temporären Verformung keratinischer Fasern hat insbesondere die Sprühapplikation entsprechender kosmetischer Zubereitungen eine große Bedeutung, wobei die Zubereitungen in der Regel als Pumpsprays oder Aerosolsprays appliziert werden. Hierzu werden die kosmetischen Zubereitungen in einer Abgabevorrichtung konfektioniert, aus denen sie entweder mittels mechanischer Krafteinwirkung oder mit Hilfe eines Treibmittels über ein Ventil versprüht werden. Beide Methoden haben offensichtliche Nachteile. Während Pumpsprays sich in der Regel nicht für eine lang anhaltende gleichmäßige Sprühapplikation haarkosmetischer Zubereitungen eignen, basieren Aersolsprays auf dem Einsatz von Treibmitteln oder Treibgasen, die einerseits keine kosmetische Wirkung entfalten und von denen andererseits bei unsachgemäßer Handhabung eine Gefährdung der Verbraucher ausgehen kann.

Vor diesem Hintergrund besteht ein Bedarf nach alternativen Wegen zur Zerstäubung haarkosmetischer Zubereitungen. Als ein solches alternatives Sprühverfahren hat sich die Entspannungsverdampfung erwiesen. Bei diesem Verfahren, das beispielsweise in der internationalen Patentanmeldung WO 2001/83071 A1 (Henkel) beschrieben wird, wird eine flüssige oder pastöse lösungsmittelhaltige Zusammensetzung in einem abgeschlossenen Raum auf eine Temperatur erhitzt, die über dem Siedepunkt des Lösungsmittels liegt, wodurch in der Zusammensetzung ein Überdruck erzeugt wird. Bei Entspannung (Drosselung) des Drucks verdampft die Flüssigkeit und kann nachfolgend beispielsweise mittels einer geeigneten Düse zerstäubt werden.

Auch wenn die Entspannungsverdampfung also grundsätzlich für die Sprühapplikation haarkosmetischer Zubereitungen geeignet ist, so kann gleichzeitig doch nicht jede haarkosmetische Zubereitung mittels eines Entspannungsverdampfungsverfahrens zerstäubt werden. Dies liegt einerseits an der für die Entspannungsverdampfung notwendigen Erhitzung der kosmetischen Zubereitung, andererseits an den Spezifika der durch Entspannungsverdampfung erzeugten Sprühnebel, beispielsweise der erzeugten Tröpfchengröße und Tröpfchendichte im Sprühnebel.

Aufgabe der vorliegenden Erfindung war es daher, spezifische haarkosmetische Zubereitungen zur temporären Verformung keratinhaltiger Fasern zur Verfügung zu stellen, welche sich aufgrund ihrer chemischen und physikalischen Eigenschaften zur zielgerichteten Sprühapplikation mittels einer spezifischen Vorrichtung zur Entspannungsverdampfung eignen. Weiterhin sollen die Zubereitungen geeignet sein, nach einer Applikation mittels eines Entspannungsverfahrens einen hohen Haltegrad, insbesondere einen hohen Langzeithaltegrad, und einen hohen Volumeneffekt zu realisieren. Es hat sich gezeigt, dass zur Lösung dieser Aufgabe aus der Vielzahl bekannten haarkosmetisch wirksamen Zubereitungen insbesondere wässrige oder wässrige/alkoholische Zubereitungen geeignet sind, die als festigende Komponente anorganisches Salz enthalten.

Ein erster Gegenstand der vorliegenden Erfindung ist somit ein kosmetisches Produkt, umfassend
a) eine kosmetische Zubereitung, enthaltend bezogen auf ihr Gesamtgewicht
   a1) 65 bis 96 Gew.-% mindestens eines polaren Lösungsmittels;
   a2) 0,1 bis 34 Gew.-% mindestens eines anorganischen Salzes;
b) eine neuerungsgemäße Vorrichtung zur Entspannungsverdampfung der kosmetischen Zubereitung a).

Die kosmetische Zubereitung a) ist dabei flüssig. Die kosmetische Zubereitung a) kann als Lösung oder Dispersion, beispielsweise als Emulsion oder Suspension vorliegen. Bevorzugte kosmetische Zubereitungen a) liegen in Form einer Lösung oder einer Suspension vor.

Die erfindungsgemäße kosmetische Zubereitung enthält als ersten wesentlichen Bestandteil 65 bis 96 Gew.-% mindestens eines polaren Lösungsmittels a1). Bevorzugte kosmetische Produkte sind dadurch gekennzeichnet, dass der Gewichtsanteil des polaren Lösungsmittels a1) am Gesamtgewicht der kosmetischen Zubereitung a) 75 bis 94 Gew.-%, vorzugsweise 85 bis 92 Gew.-% beträgt. Entsprechende Mittel zeichnen sich durch eine gute kosmetische Wirkung bei gleichzeitig guter Applizierbarkeit aus.

Zur Verbesserung der Anwendungseigenschaften erfindungsgemäßer kosmetischer Zubereitungen bei gleichzeitiger Minimierung der thermischen Belastung etwaiger Wirk- oder Hilfsstoffe im Verlauf des Entspannungsverdampfungsverfahrens, hat es sich als vorteilhaft erwiesen, polare Lösungsmittel a1) einzusetzen, welche eine Siedetemperatur (20°C, 1013 mbar) zwischen 50 und 110°C, vorzugsweise zwischen 70 und 105°C aufweisen. Als besonders geeignet haben sich dabei Ethanol, Isopropanol und Wasser erwiesen, welche aus diesem Grund als polare Lösungsmittel a1) bevorzugt werden.

Besonders bevorzugte polare Lösungsmittel a1) oder Lösungsmittelsysteme sind
- polare Lösungsmittel a1), umfassend mehr als 80 Gew.-%, vorzugsweise mehr als 88 Gew.-% und insbesondere mehr als 92 Gew.-% Wasser, jeweils bezogen auf das Gesamtgewicht des polaren Lösungsmittels;
- polare Lösungsmittel a1), umfassend, jeweils bezogen auf das Gesamtgewicht des polaren Lösungsmittels, mindestens 60 Gew.-%, bevorzugt mindestens 80 Gew.-%, besonders bevorzugt mindestens 90 Gew.-% und insbesondere mindestens 95 Gew.-% Wasser und Ethanol;
- polare Lösungsmittel a1), umfassend Wasser und Ethanol, wobei das Gewichtsverhältnis von Wasser zu Ethanol 5:1 bis 1:5, bevorzugt 2:1 bis 1:2 und insbesondere 5:4 bis 4:5 beträgt.

Ganz besonders bevorzugte kosmetische Zubereitungen sind dadurch gekennzeichnet, dass der Anteil flüchtiger Bestandteile maximal 55 Gew.-% beträgt. Zur Gruppe dieser flüchtigen Bestandteile zählt u.a. auch das polare Lösungsmittel Ethanol. Ganz besonders bevorzugte kosmetische Zubereitungen sind demnach mit anderen Worten dadurch gekennzeichnet, dass der Gewichtsanteil des Ethanols am Gesamtgewicht der kosmetischen Zubereitung maximal 55 Gew.-%, vorzugsweise 10 bis 55 Gew.-%, besonders bevorzugt 25 bis 55 Gew.-% und insbesondere 40 bis 55 Gew.-% beträgt.

Ein zweiter wesentlicher Bestandteil erfindungsgemäßer kosmetischer Zusammensetzungen ist das anorganische Salz a2). In Bezug auf die Herstellbarkeit, Applizierbarkeit und kosmetische Wirkung erfindungsgemäßer kosmetischer Zusammensetzungen hat es sich als vorteilhaft erwiesen, wenn der Gewichtsanteil des anorganischen Salzes a2) am Gesamtgewicht der kosmetischen Zubereitung a) 1,0 bis 24 Gew.-%, vorzugsweise 2,0 bis 14 Gew.-% beträgt. Besonders bevorzugt sind Salzgehalte oberhalb 3,0 Gew.-%, vorzugsweise 4,0 Gew.-% und insbesondere 5,0 Gew.-%. Bevorzugte anorganische Salze a2) sind ausgewählt aus den Chloriden und Sulfaten der Alkali- und Erdalkalimetalle. Bevorzugte Salze a2) sind beispielsweise Natriumchlorid, Natriumsulfat, Magnesiumchlorid und Magnesiumsulfat. Besonders bevorzugte Salze a2) sind Magnesiumsulfat, vorzugsweise Magnesium-Heptahydrat MgSO₄ 7 H₂O und Magnesiumchlorid MgCl2. Ein weiteres besonders bevorzugtes Salz a2) ist das Natriumchlorid NaCl.

In einer bevorzugten Ausführungsform umfasst das anorganische Salz a2) eine Kombination von Magnesiumsulfat-Heptahydrat und Natriumchlorid, wobei Gewichtsverhältnis von Magnesiumsulfat-Heptahydrat zu Natriumchlorid bevorzugt 1:2 bis 50:1, vorzugsweise 1:1 bis 25:1 und insbesondere 2:1 bis 10:1 beträgt.

Die erfindungsgemäßen kosmetischen Produkte umfassen neben der kosmetischen Zubereitung a) weiterhin eine Vorrichtung zur Entspannungsverdampfung. Der Ausdruck "Entspannungsverdampfung" bezeichnet im Rahmen der vorliegenden Anmeldung das Entstehen von Dampf bei Absenken des Druckes in einem mit Flüssigkeit befüllten, unter Überdruck (zur Umgebung) stehenden geschlossenen Raum. Ein entsprechender Überdruck lässt sich beispielsweise erzeugen, indem eine Menge der kosmetischen Zubereitung a) in einem abgeschlossenen Raum auf eine Temperatur T₁ erhitzt wird. In dem geschlossenen Raum hat die Flüssigkeit bei gegebener Temperatur T₁ einen Druck Sättigungsdruck p₁. Wird der geschlossenen Raum beispielsweise mittels eines Ventils zu einem nicht unter Überdruck stehenden Relaxationsraum mit dem Druck po < p₁ geöffnet, so sinkt der Druck in dem zuvor geschlossenen Raum ab und im Rahmen der Ausbreitung des neuen Druckniveaus verdampft die kosmetische Zubereitung a), bzw. das in der kosmetischen Zubereitung enthaltene Lösungsmittel oder Teile dieses Lösungsmittels. Der entstehende Dampf oder Sprühnebel kann für die Applikation spezifischer kosmetischer Zubereitungen genutzt werden.

Wird die kosmetische Zubereitung a) also ausgehend von Standardbedingungen (T₀ = 25°C, po = 1,000 bar) in einem geschlossenen Raum erhitzt, so resultiert neben einer erhöhten Temperatur weiterhin ein erhöhter Druck der kosmetischen Zubereitung a). Dieser erhöhte Druck kann in einem Relaxationsraum auf einen Druck po, zum Beispiel den umgebenden Luftdruck (po = 1,000 bar), entlastet werden kann, wodurch mindestens teilweise eine Verdampfung der kosmetischen Zubereitung a) erreicht wird.
Die kosmetische Zubereitung a) kann unmittelbar in dem Raum entspannt werden, in welchem sie zuvor erhitzt wurde. Die erhitzte und unter Überdruck befindliche kosmetische Zubereitung a) kann alternativ aber auch nach dem Erhitzen in einen zweiten Raum transportiert werden, in welchem dann nachfolgend die Druckentlastung erfolgt.

Bei der Entspannungsverdampfung handelt es sich mit anderen Worten um ein Verfahren, bei welchem die kosmetische Zubereitung a) in einem geschlossenen Behälter mittels einer Heizvorrichtung auf Temperaturen oberhalb der Umgebungstemperatur erhitzt wird, wobei in dem Behälter ein Druck oberhalb des Umgebungsdrucks entsteht, und die erhitzte und unter erhöhtem Druck stehende kosmetische Zubereitung a) anschließend aus dem Behälter in die Umgebung entspannt wird.

Bei einer Vorrichtung zur Entspannungsverdampfung handelt es sich demnach um eine Vorrichtung, welche einen Behälter und eine Heizvorrichtung umfasst und derart ausgestaltet ist, dass eine kosmetische Zubereitung a) in dem geschlossenen Behälter mittels der Heizvorrichtung auf Temperaturen oberhalb der Umgebungstemperatur in einer Weise erhitzt werden kann, dass in dem Behälter ein Druck oberhalb des Umgebungsdrucks entsteht und die erhitzte und unter erhöhtem Druck stehende kosmetische Zubereitung a) aus dem Behälter in die Umgebung entspannt werden kann.

Gleichzeitig mit oder nach der Druckentlastung kann die kosmetische Zubereitung a) einer Düse zugeführt werden, mittels derer beispielsweise Eigenschaften des durch die Entspannungsverdampfung erzeugten Dampfes bzw. Sprühnebels, insbesondere die Tröpfchengröße oder die Tröpfchendichte aber auch die Sprühweite und die Form des Sprühkegels beeinflusst werden können. Der Einsatz von Düsen, vorzugsweise Zerstäuberdüsen, ist daher bevorzugt. Der spezifische Düsentyp oder die spezifische Düsengestaltung wird in Abhängigkeit von den jeweiligen Sprühnebeleigenschaften gezielt festgelegt.

Zusammenfassend verfügt eine erfindungsgemäße Vorrichtung zur Entspannungsverdampfung über
b1) einen Behälter, welcher einen geschlossenen Innenraum definiert, in dem die kosmetische Zubereitung aufgenommen werden kann,
b2) ein Ventil oder ein vergleichbar wirkendes Schließelement, um den wenigstens anteilsweise mit der kosmetischen Zubereitung a) befüllten Innenraum des Behälters zu verschließen und zu öffnen,
b3) eine Heizvorrichtung, um die in dem geschlossenen Innenraum des Behälters befindliche kosmetische Zubereitung a) unter Druckerhöhung auf eine Temperatur oberhalb des Siedepunktes des in der kosmetischen Zubereitung enthaltenen polaren Lösungsmittels zu erhitzen, sowie die erhitzte kosmetische Zubereitung a) aus dem Innenraum des Behälters unter Druckminderung in die Umgebung zu entspannen,
b4) eine Düse, welche eine Zerstäubung der aus dem Behälter entweichenden kosmetischen Zubereitung a) ermöglicht.

Alternativ zu einem Ventil kann auch ein vergleichbar wirkendes Schließelement, welches eine zugehörige Öffnung im Behälter durch entsprechende Positionsänderung verschließen oder freigeben kann, zum Einsatz kommen.

Ein bevorzugter Gegenstand der vorliegenden Erfindung ist ein kosmetisches Produkt, umfassend
a) eine kosmetische Zubereitung, enthaltend bezogen auf ihr Gesamtgewicht a1) 65 bis 96 Gew.-% mindestens eines polaren Lösungsmittels; a2) 0,1 bis 34 Gew.-% mindestens eines anorganischen Salzes;
b) eine Vorrichtung zur Entspannungsverdampfung der kosmetischen Zubereitung a), mit
   b1) einem Behälter, welcher einen geschlossenen Innenraum definiert, in dem die kosmetische Zubereitung aufgenommen werden kann,
   b2) einem Ventil oder einem vergleichbar wirkenden Schließelement, um den wenigstens anteilsweise mit der kosmetischen Zubereitung a) befüllten Innenraum des Behälters zu verschließen und zu öffnen,
   b3) einer Heizvorrichtung, um die in dem geschlossenen Innenraum des Behälters befindliche kosmetische Zubereitung a) unter Druckerhöhung auf eine Temperatur oberhalb des Siedepunktes des in der kosmetischen Zubereitung enthaltenen polaren Lösungsmittels zu erhitzen, sowie die erhitzte kosmetische Zubereitung a) aus dem Innenraum des Behälters unter Druckminderung in die Umgebung zu entspannen,
   b4) einer Düse, welche eine Zerstäubung der aus dem Behälter entweichenden kosmetischen Zubereitung a) ermöglicht.

Der Behälter b1), in welchem die kosmetische Zubereitung erhitzt wird, ist in einer Weise ausgestaltet, die es ermöglicht, diesen Behälter während der Erhitzung der kosmetischen Zubereitung a) gegen die Umgebung vollständig abzuschließen und nach der Erhitzung, zur Ermöglichung Entspannungsverdampfung der kosmetischen Zubereitung a), zu öffnen. Dies kann beispielsweise durch ein Bauteil zur Durchflussregelung, insbesondere ein Ventil, gewährleistet werden.

Der Behälter b1), in welchem die kosmetische Zubereitung erhitzt wird, steht vorzugsweise in Kontakt mit einem weiteren Behälter, aus welchem die zur Entspannungsverdampfung vorgesehene Menge der kosmetischen Zubereitung vor der Erhitzung in den Behälter b1) überführt wird. Der Zugang zwischen diesem Vorratsbehälter und Behälter b1) ist dabei über eine entsprechende Vorrichtung, beispielsweise ein Ventil, zu öffnen und zu schließen. Dieser weitere Behälter ist vorzugsweise in Form eines Vorratsbehälters ausgestaltet, das heißt, er umfasst bevorzugt ein Vielfaches, beispielsweise mehr als das Zehnfache, vorzugsweise mehr als das Fünfzigfache, der für einen Verdampfungsvorgang notwendigen Menge der kosmetischen Zubereitung. Mit anderen Worten weist der weitere Behälter/Vorratsbehälter vorzugsweise ein Vielfaches, beispielsweise mehr als das Zehnfache Volumen, vorzugsweise mehr als das Zwanzigfache und insbesondere mehr als das Fünfzigfache Volumen des Behälters b1) auf.

Ein weiterer besonders bevorzugter Gegenstand der vorliegenden Erfindung ist daher ein kosmetisches Produkt, umfassend
a) eine kosmetische Zubereitung, enthaltend bezogen auf ihr Gesamtgewicht
   a1) 65 bis 96 Gew.-% mindestens eines polaren Lösungsmittels;
   a2) 0,1 bis 34 Gew.-% mindestens eines anorganischen Salzes;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   o der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   o der Vorratsbehälter mindestens das Zehnfache Volumen, vorzugsweise mindestens das Zwanzigfache und insbesondere mindestens das Fünfzigfache Volumen des Behälters b1) aufweist.

Der Vorratsbehälter ist kein Druckbehälter und die in dem Vorratsbehälter befindliche kosmetische Zusammensetzung befindet sich nicht unter Druck, mit anderen Worten entspricht der Druck im Inneren des Vorratsbehälters dem Umgebungsdruck (auch Luftdruck oder Atmosphärendruck). So umfassen entsprechende kosmetische Produkte beispielsweise keine Treibmittel. Auch verfügt das kosmetische Produkt über keine Pumpvorrichtung, die geeignet ist, die kosmetische Zubereitung ohne die Einwirkung der Vorrichtung zur Entspannungsverdampfung in die Umgebung freizusetzen oder zu versprühen.

Ein ganz besonders bevorzugter Gegenstand der vorliegenden Erfindung ist daher ein kosmetisches Produkt, umfassend
a) eine kosmetische Zubereitung, enthaltend bezogen auf ihr Gesamtgewicht
   a1) 65 bis 96 Gew.-% mindestens eines polaren Lösungsmittels;
   a2) 0,1 bis 34 Gew.-% mindestens eines anorganischen Salzes;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   o der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   o der Druck im Inneren des Vorratsbehälters dem Umgebungsdruck entspricht.

Ein ganz besonders bevorzugter Gegenstand der vorliegenden Erfindung ist daher ein kosmetisches Produkt, umfassend
a) eine kosmetische Zubereitung, enthaltend bezogen auf ihr Gesamtgewicht
   a1) 65 bis 96 Gew.-% mindestens eines polaren Lösungsmittels;
   a2) 0,1 bis 34 Gew.-% mindestens eines anorganischen Salzes;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   o der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   o der Druck im Inneren des Vorratsbehälters dem Umgebungsdruck entspricht und das kosmetische Produkt kein Treibmittel umfasst.

Bevorzugt werden weiterhin kosmetische Produkte, umfassend
a) eine kosmetische Zubereitung, enthaltend bezogen auf ihr Gesamtgewicht
   a1) 65 bis 96 Gew.-% mindestens eines polaren Lösungsmittels;
   a2) 0,1 bis 34 Gew.-% mindestens eines anorganischen Salzes;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   o der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   o der Druck im Inneren des Vorratsbehälters dem Umgebungsdruck entspricht,
wobei das kosmetische Produkt keine Pumpvorrichtung aufweist, die geeignet ist, die kosmetische Zubereitung a) ohne die Einwirkung der Vorrichtung zur Entspannungsverdampfung freizusetzen oder zu versprühen.

Zusammenfassend ist ein besonders bevorzugter Gegenstand der vorliegenden Erfindung daher ein kosmetisches Produkt, umfassend
a) eine kosmetische Zubereitung, enthaltend bezogen auf ihr Gesamtgewicht
   a1) 65 bis 96 Gew.-% mindestens eines polaren Lösungsmittels;
   a2) 0,1 bis 34 Gew.-% mindestens eines anorganischen Salzes;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   o der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   o der Druck im Inneren des Vorratsbehälters dem Umgebungsdruck entspricht und das kosmetische Produkt kein Treibmittel umfasst,
wobei das kosmetische Produkt keine Pumpvorrichtung aufweist, die geeignet ist, die kosmetische Zubereitung a) ohne die Einwirkung der Vorrichtung zur Entspannungsverdampfung freizusetzen oder zu versprühen.

Neben den beiden zuvor beschriebenen Bestandteilen a1) und a2) können die erfindungsgemäßen kosmetischen Zubereitungen a) weitere Wirk- oder Hilfsstoffe enthalten, wobei insbesondere solche Wirk- oder Hilfsstoffe bevorzugt werden, welche die Herstellbarkeit, Applizierbarkeit und/oder kosmetische Wirkung erfindungsgemäßer kosmetischer Zubereitungen verbessern.

Zur Verbesserung der Herstellbarkeit, Applizierbarkeit und kosmetischen Wirkung enthält die kosmetische Zubereitung a) vorzugsweise nichtionisches Tensid a3), wobei besonders bevorzugte kosmetische Zubereitungen a) dadurch gekennzeichnet sind, dass sie bezogen auf ihr Gesamtgewicht 0,05 bis 4,0 Gew.-%, vorzugsweise 0,1 bis 2,0 Gew.-% und insbesondere 0,2 bis 1,0 Gew.-% nichtionisches Tensid a3) enthalten.

Bevorzugte nichtionische Tenside sind PEG-Derivate von hydriertem Ricinusöl, die z. B. unter der Bezeichnung PEG Hydrogenated Castor Oil erhältlich sind, z.B. PEG-30 Hydrogenated Castor Oil, PEG-33 Hydrogenated Castor Oil, PEG-35 Hydrogenated Castor Oil, PEG-36 Hydrogenated Castor Oil, PEG-40 Hydrogenated Castor Oil oder PEG-60 Hydrogenated Castor Oil. Erfindungsgemäß besonders bevorzugt sind nichtionische Tenside ausgewählt aus der Gruppe der PEG-Derivate von hydriertem Ricinusöl, besonders bevorzugt aus der Gruppe PEG-40 Hydrogenated Castor Oil und PEG-60 Hydrogenated Castor Oil enthält.

Eine zweite Gruppe bevorzugter Inhaltsstoffe kosmetischer Zubereitungen a) bilden die Polyalkylenglycole a4), insbesondere die Polyethylenglycole. Für die kosmetische Wirkung erfindungsgemäßer Zubereitungen hat es sich als vorteilhaft erwiesen, wenn diese, bezogen auf ihr Gesamtgewicht, 0,1 bis 10 Gew.-%, vorzugsweise 0,2 bis 8,0 Gew.-%, und insbesondere 0,5 bis 5,0 Gew.-% eines Polyalkylenglycols a4) enthält. Zur Gruppe der Polyalkylenglycole zählen beispielsweise die Polyethylenglycole und die besonders bevorzugten Polypropylenglycole.

Erfindungsgemäß geeignet sind flüssige wie feste Polyethylenglykole (PEG), beispielsweise Polyalkylenglykole mit Molmassen von 250 (PEG-4) bis 3350 (PEG-75). Besonders bevorzugt werden Polyethylenglycole mit der INCI-Bezeichnung PEG-6, PEG-7, PEG-8, PEG-9, PEG-10, PEG-12, PEG-14, PEG-16, PEG-18, PEG-20, PEG-32 und PEG-40. Insbesondere bevorzugt sind die Polyethylenglycole mit der INCI-Bezeichnung PEG-8 und PEG-32.

Eine dritte Gruppe bevorzugter Wirk- und Hilfsstoffe bilden die filmbildenden Polymere a5), deren Gewichtsanteil am Gesamtgewicht der kosmetischen Zubereitung a) im Falle eines Einsatzes 0,1 bis 10 Gew.-%, vorzugsweise 0,2 bis 8,0 Gew.-%, und insbesondere 0,5 bis 5,0 Gew.-% beträgt.

Als filmbildende Polymere a5) eignen sich permanent als auch temporär kationische, anionische, nichtionische oder amphotere Polymere. Diese filmbildenden Polymere können synthetischen oder natürlichen Ursprungs sein.

Beispiele für gebräuchliche filmbildende Polymere a5) sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/ Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate und Styrene/VP Copolymer.

Eine erste Gruppe besonders bevorzugter Polymere a5) bilden die Vinylpyrrolidon Homo- oder Copolymere. Mit besonderem Vorzug eingesetzte Polymere sind:
- Polyvinylpyrrolidone (INCI-Bezeichnung PVP; CAS-Nummer 9003-39-8), wie sie beispielsweise unter der Bezeichnung Luviskol® (BASF) vertrieben werden;
- Copolymere von Vinylpyrrolidon und Vinylacetat (INCI-Bezeichnung VP/VA Copolymer; CAS-Nummer: 25086-89-9), wie sie beispielsweise unter der Bezeichnung Luviskol® VA (BASF) vertrieben werden;
- Copolymere von Vinylpyrrolidon und Dimethylaminopropylmethacrylamid (INCI-Bezeichnung: VP/DMAPAAcrylates Copolymer; CAS-Nummer: 175893-71-7), wie sie beispielsweise unterder Bezeichnung Styleze® CC-10 (Ashland) vertrieben werden;
- Copolymere von Vinylpyrrolidon mit Vinylcaprolactam und Dimethylaminoethylmethacrylat (INCI-Bezeichnung: Vinyl Caprolactam/VP/Dimethylaminoethyl Methacrylate Copolymer; CAS-Nummer: 102972-64-5), wie sie beispielsweise unter der Bezeichnung Advantage® LC (Ashland) vertrieben werden.

Aufgrund ihrer kosmetischen Wirkung in Kombination mit den anorganischen Salzen a2) erfindungsgemäß bevorzugt eingesetzte filmbildenden Polymere sind insbesondere ausgewählt aus der Gruppe der Vinylpyrrolidion Homo- und Copolymere, vorzugsweise aus der Gruppe der Polyvinylpyrrolidone und der Copolymere von Vinylpyrrolidon und Vinylacetat, vorzugsweise der Vinylpyrrolidon/Vinylacetat -Copolymere.

Als weitere geeignete Wirk- oder Hilfsstoffe sind insbesondere zusätzliche Pflegestoffe zu nennen.

Als Pflegestoff kann das Mittel beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Weitere Pflegestoffe sind Panthenol, Coffein, Nicotinamid und Sorbitol.

Als Pflegestoff können die erfindungsgemäßen Mittel weiterhin mindestens einen Pflanzenextrakt, aber auch Mono- bzw. Oligosaccharide und/oder Lipide enthalten.

Die Zusammensetzung einiger besonders bevorzugter erfindungsgemäßer kosmetischer Zubereitungen kann den folgenden Tabellen entnommen werden (Angaben in Gew.-% bezogen auf das Gesamtgewicht des kosmetischen Mittels sofern nicht anders angegeben). Bezüglich weiterer bevorzugter Ausführungsformen dieser besonders bevorzugten Zusammensetzungen gilt mutatis mutandis das zuvor zu den erfindungsgemäßen kosmetischen Zubereitungen a) Gesagte.

| | Formel 1 | Formel 2 | Formel 3 | Formel 4 | Formel 5 |
|---|---|---|---|---|---|
| polares Lösungsmittel a1) | 65 bis 96 | 75 bis 94 | 85 bis 92 | 88 | 91 |
| anorganisches Salz a2) | 0,1 bis 34 | 1,0 bis 24 | 2,0 bis 14 | 11 | 3,0 |
| optionale Additive | add 100 | add 100 | add 100 | add 100 | add 100 |
| | | | | | |

| | Formel 6 | Formel 7 | Formel 8 | Formel 9 | Formel 10 |
|---|---|---|---|---|---|
| Wasser | 65 bis 96 | 75 bis 94 | 85 bis 92 | 88 | 91 |
| anorganisches Salz a2) | 0,1 bis 34 | 1,0 bis 24 | 2,0 bis 14 | 11 | 3,0 |
| optionale Additive | add 100 | add 100 | add 100 | add 100 | add 100 |
| | | | | | |

| | Formel 11 | Formel 12 | Formel 13 | Formel 14 | Formel 15 |
|---|---|---|---|---|---|
| Wasser, Ethanol | 65 bis 96 | 75 bis 94 | 85 bis 92 | 88 | 91 |
| anorganisches Salz a2) | 0,1 bis 34 | 1,0 bis 24 | 2,0 bis 14 | 11 | 3,0 |
| optionale Additive | add 100 | add 100 | add 100 | add 100 | add 100 |
| | | | | | |

| | Formel 16 | Formel 17 | Formel 18 | Formel 19 | Formel 20 |
|---|---|---|---|---|---|
| polares Lösungsmittel a1) | 65 bis 96 | 75 bis 94 | 85 bis 92 | 88 | 91 |
| Natriumchlorid | 0,1 bis 34 | 1,0 bis 24 | 2,0 bis 14 | 11 | 3,0 |
| optionale Additive | add 100 | add 100 | add 100 | add 100 | add 100 |
| | | | | | |

| | Formel 21 | Formel 22 | Formel 23 | Formel 24 | Formel 25 |
|---|---|---|---|---|---|
| Wasser | 65 bis 96 | 75 bis 94 | 85 bis 92 | 88 | 91 |
| Natriumchlorid | 0,1 bis 34 | 1,0 bis 24 | 2,0 bis 14 | 11 | 3,0 |
| optionale Additive | add 100 | add 100 | add 100 | add 100 | add 100 |
| | | | | | |

| | Formel 26 | Formel 27 | Formel 28 | Formel 29 | Formel 30 |
|---|---|---|---|---|---|
| Wasser, Ethanol | 65 bis 96 | 75 bis 94 | 85 bis 92 | 88 | 91 |
| Natriumchlorid | 0,1 bis 34 | 1,0 bis 24 | 2,0 bis 14 | 11 | 3,0 |
| optionale Additive | add 100 | add 100 | add 100 | add 100 | add 100 |
| | | | | | |

| | Formel 31 | Formel 32 | Formel 33 | Formel 34 | Formel 35 |
|---|---|---|---|---|---|
| polares Lösungsmittel a1) | 65 bis 96 | 75 bis 94 | 85 bis 92 | 88 | 91 |
| Magnesiumsulfat | 0,1 bis 34 | 1,0 bis 24 | 2,0 bis 14 | 11 | 3,0 |
| optionale Additive | add 100 | add 100 | add 100 | add 100 | add 100 |
| | | | | | |

| | Formel 36 | Formel 37 | Formel 38 | Formel 39 | Formel 40 |
|---|---|---|---|---|---|
| Wasser | 65 bis 96 | 75 bis 94 | 85 bis 92 | 88 | 91 |
| Magnesiumsulfat | 0,1 bis 34 | 1,0 bis 24 | 2,0 bis 14 | 11 | 3,0 |
| optionale Additive | add 100 | add 100 | add 100 | add 100 | add 100 |
| | | | | | |

| | Formel 41 | Formel 42 | Formel 43 | Formel 44 | Formel 45 |
|---|---|---|---|---|---|
| Wasser, Ethanol | 65 bis 96 | 75 bis 94 | 85 bis 92 | 88 | 91 |
| Magnesiumsulfat | 0,1 bis 34 | 1,0 bis 24 | 2,0 bis 14 | 11 | 3,0 |
| optionale Additive | add 100 | add 100 | add 100 | add 100 | add 100 |
| | | | | | |

| | Formel 46 | Formel 47 | Formel 48 | Formel 49 | Formel 50 |
|---|---|---|---|---|---|
| polares Lösungsmittel a1) | 65 bis 96 | 75 bis 94 | 85 bis 92 | 88 | 91 |
| Natriumchlorid, Magnesiumsulfat | 0,1 bis 34 | 1,0 bis 24 | 2,0 bis 14 | 11 | 3,0 |
| optionale Additive | add 100 | add 100 | add 100 | add 100 | add 100 |
| | | | | | |

| | Formel 51 | Formel 52 | Formel 53 | Formel 54 | Formel 55 |
|---|---|---|---|---|---|
| Wasser | 65 bis 96 | 75 bis 94 | 85 bis 92 | 88 | 91 |
| Natriumchlorid, Magnesiumsulfat | 0,1 bis 34 | 1,0 bis 24 | 2,0 bis 14 | 11 | 3,0 |
| optionale Additive | add 100 | add 100 | add 100 | add 100 | add 100 |
| | | | | | |

| | Formel 56 | Formel 57 | Formel 58 | Formel 59 | Formel 60 |
|---|---|---|---|---|---|
| Wasser, Ethanol | 65 bis 96 | 75 bis 94 | 85 bis 92 | 88 | 91 |
| Natriumchlorid, Magnesiumsulfat | 0,1 bis 34 | 1,0 bis 24 | 2,0 bis 14 | 11 | 3,0 |
| optionale Additive | add 100 | add 100 | add 100 | add 100 | add 100 |
| | | | | | |

| | Formel 61 | Formel 62 | Formel 63 | Formel 64 | Formel 65 |
|---|---|---|---|---|---|
| polares Lösungsmittel a1) | 65 bis 96 | 75 bis 94 | 85 bis 92 | 88 | 91 |
| anorganisches Salz a2) | 0,1 bis 34 | 1,0 bis 24 | 2,0 bis 14 | 11 | 3,0 |
| nichtionisches Tensid a3) | 0,05 bis 4,0 | 0,1 bis 2,0 | 0,2 bis 1,0 | 0,2 | 0,4 |
| optionale Additive | add 100 | add 100 | add 100 | add 100 | add 100 |
| | | | | | |

| | Formel 66 | Formel 67 | Formel 68 | Formel 69 | Formel 70 |
|---|---|---|---|---|---|
| Wasser | 65 bis 96 | 75 bis 94 | 85 bis 92 | 88 | 91 |
| anorganisches Salz a2) | 0,1 bis 34 | 1,0 bis 24 | 2,0 bis 14 | 11 | 3,0 |
| nichtionisches Tensid a3) | 0,05 bis 4,0 | 0,1 bis 2,0 | 0,2 bis 1,0 | 0,2 | 0,4 |
| optionale Additive | add 100 | add 100 | add 100 | add 100 | add 100 |
| | | | | | |

| | Formel 71 | Formel 72 | Formel 73 | Formel 74 | Formel 75 |
|---|---|---|---|---|---|
| Wasser, Ethanol | 65 bis 96 | 75 bis 94 | 85 bis 92 | 88 | 91 |
| anorganisches Salz a2) | 0,1 bis 34 | 1,0 bis 24 | 2,0 bis 14 | 11 | 3,0 |
| nichtionisches Tensid a3) | 0,05 bis 4,0 | 0,1 bis 2,0 | 0,2 bis 1,0 | 0,2 | 0,4 |
| optionale Additive | add 100 | add 100 | add 100 | add 100 | add 100 |
| | | | | | |

| | Formel 76 | Formel 77 | Formel 78 | Formel 79 | Formel 80 |
|---|---|---|---|---|---|
| polares Lösungsmittel a1) | 65 bis 96 | 75 bis 94 | 85 bis 92 | 88 | 91 |
| Natriumchlorid | 0,1 bis 34 | 1,0 bis 24 | 2,0 bis 14 | 11 | 3,0 |
| nichtionisches Tensid a3) | 0,05 bis 4,0 | 0,1 bis 2,0 | 0,2 bis 1,0 | 0,2 | 0,4 |
| optionale Additive | add 100 | add 100 | add 100 | add 100 | add 100 |
| | | | | | |

| | Formel 81 | Formel 82 | Formel 83 | Formel 84 | Formel 85 |
|---|---|---|---|---|---|
| Wasser | 65 bis 96 | 75 bis 94 | 85 bis 92 | 88 | 91 |
| Natriumchlorid | 0,1 bis 34 | 1,0 bis 24 | 2,0 bis 14 | 11 | 3,0 |
| nichtionisches Tensid a3) | 0,05 bis 4,0 | 0,1 bis 2,0 | 0,2 bis 1,0 | 0,2 | 0,4 |
| optionale Additive | add 100 | add 100 | add 100 | add 100 | add 100 |
| | | | | | |

| | Formel 86 | Formel 87 | Formel 88 | Formel 89 | Formel 90 |
|---|---|---|---|---|---|
| Wasser, Ethanol | 65 bis 96 | 75 bis 94 | 85 bis 92 | 88 | 91 |
| Natriumchlorid | 0,1 bis 34 | 1,0 bis 24 | 2,0 bis 14 | 11 | 3,0 |
| nichtionisches Tensid a3) | 0,05 bis 4,0 | 0,1 bis 2,0 | 0,2 bis 1,0 | 0,2 | 0,4 |
| optionale Additive | add 100 | add 100 | add 100 | add 100 | add 100 |
| | | | | | |

| | Formel 91 | Formel 92 | Formel 93 | Formel 94 | Formel 95 |
|---|---|---|---|---|---|
| polares Lösungsmittel a1) | 65 bis 96 | 75 bis 94 | 85 bis 92 | 88 | 91 |
| Magnesiumsulfat | 0,1 bis 34 | 1,0 bis 24 | 2,0 bis 14 | 11 | 3,0 |
| nichtionisches Tensid a3) | 0,05 bis 4,0 | 0,1 bis 2,0 | 0,2 bis 1,0 | 0,2 | 0,4 |
| optionale Additive | add 100 | add 100 | add 100 | add 100 | add 100 |
| | | | | | |

| | Formel 96 | Formel 97 | Formel 98 | Formel 99 | Formel 100 |
|---|---|---|---|---|---|
| Wasser | 65 bis 96 | 75 bis 94 | 85 bis 92 | 88 | 91 |
| Magnesiumsulfat | 0,1 bis 34 | 1,0 bis 24 | 2,0 bis 14 | 11 | 3,0 |
| nichtionisches Tensid a3) | 0,05 bis 4,0 | 0,1 bis 2,0 | 0,2 bis 1,0 | 0,2 | 0,4 |
| optionale Additive | add 100 | add 100 | add 100 | add 100 | add 100 |
| | | | | | |

| | Formel 101 | Formel 102 | Formel 103 | Formel 104 | Formel 105 |
|---|---|---|---|---|---|
| Wasser, Ethanol | 65 bis 96 | 75 bis 94 | 85 bis 92 | 88 | 91 |
| Magnesiumsulfat | 0,1 bis 34 | 1,0 bis 24 | 2,0 bis 14 | 11 | 3,0 |
| nichtionisches Tensid a3) | 0,05 bis 4,0 | 0,1 bis 2,0 | 0,2 bis 1,0 | 0,2 | 0,4 |
| optionale Additive | add 100 | add 100 | add 100 | add 100 | add 100 |
| | | | | | |

| | Formel 106 | Formel 107 | Formel 108 | Formel 109 | Formel 110 |
|---|---|---|---|---|---|
| polares Lösungsmittel a1) | 65 bis 96 | 75 bis 94 | 85 bis 92 | 88 | 91 |
| Natriumchlorid, Magnesiumsulfat | 0,1 bis 34 | 1,0 bis 24 | 2,0 bis 14 | 11 | 3,0 |
| nichtionisches Tensid a3) | 0,05 bis 4,0 | 0,1 bis 2,0 | 0,2 bis 1,0 | 0,2 | 0,4 |
| optionale Additive | add 100 | add 100 | add 100 | add 100 | add 100 |
| | | | | | |

| | Formel 111 | Formel 112 | Formel 113 | Formel 114 | Formel 115 |
|---|---|---|---|---|---|
| Wasser | 65 bis 96 | 75 bis 94 | 85 bis 92 | 88 | 91 |
| Natriumchlorid, Magnesiumsulfat | 0,1 bis 34 | 1,0 bis 24 | 2,0 bis 14 | 11 | 3,0 |
| nichtionisches Tensid a3) | 0,05 bis 4,0 | 0,1 bis 2,0 | 0,2 bis 1,0 | 0,2 | 0,4 |
| optionale Additive | add 100 | add 100 | add 100 | add 100 | add 100 |
| | | | | | |

| | Formel 116 | Formel 117 | Formel 118 | Formel 119 | Formel 120 |
|---|---|---|---|---|---|
| Wasser, Ethanol | 65 bis 96 | 75 bis 94 | 85 bis 92 | 88 | 91 |
| Natriumchlorid, Magnesiumsulfat | 0,1 bis 34 | 1,0 bis 24 | 2,0 bis 14 | 11 | 3,0 |
| nichtionisches Tensid a3) | 0,05 bis 4,0 | 0,1 bis 2,0 | 0,2 bis 1,0 | 0,2 | 0,4 |
| optionale Additive | add 100 | add 100 | add 100 | add 100 | add 100 |
| | | | | | |

| | Formel 121 | Formel 122 | Formel 123 | Formel 124 | Formel 125 |
|---|---|---|---|---|---|
| polares Lösungsmittel a1) | 65 bis 96 | 75 bis 94 | 85 bis 92 | 88 | 91 |
| anorganisches Salz a2) | 0,1 bis 34 | 1,0 bis 24 | 2,0 bis 14 | 11 | 3,0 |
| Polyalkylenglycol a4) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 126 | Formel 127 | Formel 128 | Formel 129 | Formel 130 |
|---|---|---|---|---|---|
| Wasser | 65 bis 96 | 75 bis 94 | 85 bis 92 | 88 | 91 |
| anorganisches Salz a2) | 0,1 bis 34 | 1,0 bis 24 | 2,0 bis 14 | 11 | 3,0 |
| Polyalkylenglycol a4) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 131 | Formel 132 | Formel133 | Formel 134 | Formel 135 |
|---|---|---|---|---|---|
| Wasser, Ethanol | 65 bis 96 | 75 bis 94 | 85 bis 92 | 88 | 91 |
| anorganisches Salz a2) | 0,1 bis 34 | 1,0 bis 24 | 2,0 bis 14 | 11 | 3,0 |
| Polyalkylenglycol a4) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel136 | Formel 137 | Formel138 | Formel 139 | Formel 140 |
|---|---|---|---|---|---|
| polares Lösungsmittel a1) | 65 bis 96 | 75 bis 94 | 85 bis 92 | 88 | 91 |
| Natriumchlorid | 0,1 bis 34 | 1,0 bis 24 | 2,0 bis 14 | 11 | 3,0 |
| Polyalkylenglycol a4) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 141 | Formel 142 | Formel 143 | Formel 144 | Formel 145 |
|---|---|---|---|---|---|
| Wasser | 65 bis 96 | 75 bis 94 | 85 bis 92 | 88 | 91 |
| Natriumchlorid | 0,1 bis 34 | 1,0 bis 24 | 2,0 bis 14 | 11 | 3,0 |
| Polyalkylenglycol a4) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel146 | Formel 147 | Formel148 | Formel 149 | Formel 150 |
|---|---|---|---|---|---|
| Wasser, Ethanol | 65 bis 96 | 75 bis 94 | 85 bis 92 | 88 | 91 |
| Natriumchlorid | 0,1 bis 34 | 1,0 bis 24 | 2,0 bis 14 | 11 | 3,0 |
| Polyalkylenglycol a4) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 151 | Formel 152 | Formel 153 | Formel 154 | Formel 155 |
|---|---|---|---|---|---|
| polares Lösungsmittel a1) | 65 bis 96 | 75 bis 94 | 85 bis 92 | 88 | 91 |
| Magnesiumsulfat | 0,1 bis 34 | 1,0 bis 24 | 2,0 bis 14 | 11 | 3,0 |
| Polyalkylenglycol a4) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 156 | Formel 157 | Formel 158 | Formel 159 | Formel 160 |
|---|---|---|---|---|---|
| Wasser | 65 bis 96 | 75 bis 94 | 85 bis 92 | 88 | 91 |
| Magnesiumsulfat | 0,1 bis 34 | 1,0 bis 24 | 2,0 bis 14 | 11 | 3,0 |
| Polyalkylenglycol a4) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 161 | Formel 162 | Formel 163 | Formel 164 | Formel 165 |
|---|---|---|---|---|---|
| Wasser, Ethanol | 65 bis 96 | 75 bis 94 | 85 bis 92 | 88 | 91 |
| Magnesiumsulfat | 0,1 bis 34 | 1,0 bis 24 | 2,0 bis 14 | 11 | 3,0 |
| Polyalkylenglycol a4) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 166 | Formel 167 | Formel 168 | Formel 169 | Formel 170 |
|---|---|---|---|---|---|
| polares Lösungsmittel a1) | 65 bis 96 | 75 bis 94 | 85 bis 92 | 88 | 91 |
| Natriumchlorid, Magnesiumsulfat | 0,1 bis 34 | 1,0 bis 24 | 2,0 bis 14 | 11 | 3,0 |
| Polyalkylenglycol a4) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 171 | Formel 172 | Formel 173 | Formel 174 | Formel 175 |
|---|---|---|---|---|---|
| Wasser | 65 bis 96 | 75 bis 94 | 85 bis 92 | 88 | 91 |
| Natriumchlorid, Magnesiumsulfat | 0,1 bis 34 | 1,0 bis 24 | 2,0 bis 14 | 11 | 3,0 |
| Polyalkylenglycol a4) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 176 | Formel 177 | Formel 178 | Formel 179 | Formel 180 |
|---|---|---|---|---|---|
| Wasser, Ethanol | 65 bis 96 | 75 bis 94 | 85 bis 92 | 88 | 91 |
| Natriumchlorid, Magnesiumsulfat | 0,1 bis 34 | 1,0 bis 24 | 2,0 bis 14 | 11 | 3,0 |
| Polyalkylenglycol a4) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 181 | Formel 182 | Formel 183 | Formel 184 | Formel 185 |
|---|---|---|---|---|---|
| polares Lösungsmittel a1) | 65 bis 96 | 75 bis 94 | 85 bis 92 | 88 | 91 |
| anorganisches Salz a2) | 0,1 bis 34 | 1,0 bis 24 | 2,0 bis 14 | 11 | 3,0 |
| filmbildendes Polymer a5) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 186 | Formel 187 | Formel 188 | Formel 189 | Formel 190 |
|---|---|---|---|---|---|
| Wasser | 65 bis 96 | 75 bis 94 | 85 bis 92 | 88 | 91 |
| anorganisches Salz a2) | 0,1 bis 34 | 1,0 bis 24 | 2,0 bis 14 | 11 | 3,0 |
| filmbildendes Polymer a5) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 191 | Formel 192 | Formel 193 | Formel 194 | Formel 195 |
|---|---|---|---|---|---|
| Wasser, Ethanol | 65 bis 96 | 75 bis 94 | 85 bis 92 | 88 | 91 |
| anorganisches Salz a2) | 0,1 bis 34 | 1,0 bis 24 | 2,0 bis 14 | 11 | 3,0 |
| filmbildendes Polymer a5) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 196 | Formel 197 | Formel 198 | Formel 199 | Formel 200 |
|---|---|---|---|---|---|
| polares Lösungsmittel a1) | 65 bis 96 | 75 bis 94 | 85 bis 92 | 88 | 91 |
| Natriumchlorid | 0,1 bis 34 | 1,0 bis 24 | 2,0 bis 14 | 11 | 3,0 |
| filmbildendes Polymer a5) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 201 | Formel 202 | Formel 203 | Formel 204 | Formel 205 |
|---|---|---|---|---|---|
| Wasser | 65 bis 96 | 75 bis 94 | 85 bis 92 | 88 | 91 |
| Natriumchlorid | 0,1 bis 34 | 1,0 bis 24 | 2,0 bis 14 | 11 | 3,0 |
| filmbildendes Polymer a5) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 206 | Formel 207 | Formel 208 | Formel 209 | Formel 210 |
|---|---|---|---|---|---|
| Wasser, Ethanol | 65 bis 96 | 75 bis 94 | 85 bis 92 | 88 | 91 |
| Natriumchlorid | 0,1 bis 34 | 1,0 bis 24 | 2,0 bis 14 | 11 | 3,0 |
| filmbildendes Polymer a5) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 211 | Formel 212 | Formel 213 | Formel 214 | Formel 215 |
|---|---|---|---|---|---|
| polares Lösungsmittel a1) | 65 bis 96 | 75 bis 94 | 85 bis 92 | 88 | 91 |
| Magnesiumsulfat | 0,1 bis 34 | 1,0 bis 24 | 2,0 bis 14 | 11 | 3,0 |
| filmbildendes Polymer a5) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 216 | Formel 217 | Formel 218 | Formel 219 | Formel 220 |
|---|---|---|---|---|---|
| Wasser | 65 bis 96 | 75 bis 94 | 85 bis 92 | 88 | 91 |
| Magnesiumsulfat | 0,1 bis 34 | 1,0 bis 24 | 2,0 bis 14 | 11 | 3,0 |
| filmbildendes Polymer a5) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 221 | Formel 222 | Formel 223 | Formel 224 | Formel 225 |
|---|---|---|---|---|---|
| Wasser, Ethanol | 65 bis 96 | 75 bis 94 | 85 bis 92 | 88 | 91 |
| Magnesiumsulfat | 0,1 bis 34 | 1,0 bis 24 | 2,0 bis 14 | 11 | 3,0 |
| filmbildendes Polymer a5) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 226 | Formel 227 | Formel 228 | Formel 229 | Formel 230 |
|---|---|---|---|---|---|
| polares Lösungsmittel a1) | 65 bis 96 | 75 bis 94 | 85 bis 92 | 88 | 91 |
| Natriumchlorid, Magnesiumsulfat | 0,1 bis 34 | 1,0 bis 24 | 2,0 bis 14 | 11 | 3,0 |
| filmbildendes Polymer a5) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 231 | Formel 232 | Formel 233 | Formel 234 | Formel 235 |
|---|---|---|---|---|---|
| Wasser | 65 bis 96 | 75 bis 94 | 85 bis 92 | 88 | 91 |
| Natriumchlorid, Magnesiumsulfat | 0,1 bis 34 | 1,0 bis 24 | 2,0 bis 14 | 11 | 3,0 |
| filmbildendes Polymer a5) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 236 | Formel 237 | Formel 238 | Formel 239 | Formel 240 |
|---|---|---|---|---|---|
| Wasser, Ethanol | 65 bis 96 | 75 bis 94 | 85 bis 92 | 88 | 91 |
| Natriumchlorid, Magnesiumsulfat | 0,1 bis 34 | 1,0 bis 24 | 2,0 bis 14 | 11 | 3,0 |
| filmbildendes Polymer a5) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 241 | Formel 242 | Formel 243 | Formel 244 | Formel 245 |
|---|---|---|---|---|---|
| polares Lösungsmittel a1) | 65 bis 96 | 75 bis 94 | 85 bis 92 | 88 | 91 |
| anorganisches Salz a2) | 0,1 bis 34 | 1,0 bis 24 | 2,0 bis 14 | 11 | 3,0 |
| nichtionisches Tensid a3) | 0,05 bis 4,0 | 0,1 bis 2,0 | 0,2 bis 1,0 | 0,2 | 0,4 |
| Polyalkylenglycol a4) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 246 | Formel 247 | Formel 248 | Formel 249 | Formel 250 |
|---|---|---|---|---|---|
| polares Lösungsmittel a1) | 65 bis 96 | 75 bis 94 | 85 bis 92 | 88 | 91 |
| anorganisches Salz a2) | 0,1 bis 34 | 1,0 bis 24 | 2,0 bis 14 | 11 | 3,0 |
| nichtionisches Tensid a3) | 0,05 bis 4,0 | 0,1 bis 2,0 | 0,2 bis 1,0 | 0,2 | 0,4 |
| filmbildendes Polymer a5) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 251 | Formel 252 | Formel 253 | Formel 254 | Formel 255 |
|---|---|---|---|---|---|
| polares Lösungsmittel a1) | 65 bis 96 | 75 bis 94 | 85 bis 92 | 88 | 91 |
| anorganisches Salz a2) | 0,1 bis 34 | 1,0 bis 24 | 2,0 bis 14 | 11 | 3,0 |
| Polyalkylenglycol a4) | 0,1 bis 10 | 0,2 bis 8,0 | 0,5 bis 5,0 | 0,2 | 2,0 |
| filmbildendes Polymer a5) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 256 | Formel 257 | Formel 258 | Formel 259 | Formel 260 |
|---|---|---|---|---|---|
| polares Lösungsmittel a1) | 65 bis 96 | 75 bis 94 | 85 bis 92 | 88 | 91 |
| anorganisches Salz a2) | 0,1 bis 34 | 1,0 bis 24 | 2,0 bis 14 | 11 | 3,0 |
| nichtionisches Tensid a3) | 0,05 bis 4,0 | 0,1 bis 2,0 | 0,2 bis 1,0 | 0,2 | 0,4 |
| Polyalkylenglycol a4) | 0,1 bis 10 | 0,2 bis 8,0 | 0,5 bis 5,0 | 0,2 | 2,0 |
| filmbildendes Polymer a5) | 0,1 bis 10 | 0,2 bis 8,0 | 0,5 bis 5,0 | 0,2 | 2,0 |
| optionale Additive | add 100 | add 100 | add 100 | add 100 | add 100 |
| | | | | | |

| | Formel 261 | Formel 262 | Formel 263 | Formel 264 | Formel 265 |
|---|---|---|---|---|---|
| Wasser | 65 bis 96 | 75 bis 94 | 85 bis 92 | 88 | 91 |
| Magnesiumsulfat | 0,1 bis 34 | 1,0 bis 24 | 2,0 bis 14 | 11 | 3,0 |
| PEG-40 Hydrogenated Castor Oil | 0,05 bis 4,0 | 0,1 bis 2,0 | 0,2 bis 1,0 | 0,2 | 0,4 |
| PEG-32 | 0,1 bis 10 | 0,2 bis 8,0 | 0,5 bis 5,0 | 0,2 | 2,0 |
| PVP/VA Copolymer | 0,1 bis 10 | 0,2 bis 8,0 | 0,5 bis 5,0 | 0,2 | 2,0 |
| optionale Additive | add 100 | add 100 | add 100 | add 100 | add 100 |

Ganz besonders bevorzugte kosmetische Zubereitungen enthalten neben den zuvor beschriebenen Bestandteilen a1) bis a5) nur geringe Mengen weiterer Wirk- und Hilfsstoffe. Kosmetische Zubereitungen, dadurch gekennzeichnet, dass der Gewichtsanteil der Bestandteile a1), a2) und, sofern vorhanden, a3), a4) und/oder a5) am Gesamtgewicht der kosmetischen Zubereitung mindestens 80 Gew.-%, vorzugsweise mindestens 90 Gew.-%, besonders bevorzugt mindestens 95 Gew.-% beträgt, sind aufgrund ihrer einfachen Herstellbarkeit und guten kosmetischen Wirkung besonders bevorzugt. Ganz besonders bevorzugte kosmetische Zubereitungen bestehen, bezogen auf ihr Gesamtgewicht, zu mindestens 84 Gew.-%, vorzugsweise mindestens 88 Gew.-% und insbesondere mindestens 92 Gew.-% aus den Bestanteilen a1) und a2).

Wie eingangs ausgeführt eignen sich die erfindungsgemäßen kosmetischen Zubereitungen a) in besonderer Weise für die Applikation mittels einer Vorrichtung zur Entspannungsverdampfung. Ein weiterer Gegenstand der vorliegenden Anmeldung ist daher die Verwendung einer kosmetischen Zubereitung a) enthaltend, bezogen auf ihr Gesamtgewicht,
a1) 65 bis 96 Gew.-% mindestens eines polaren Lösungsmittels;
a2) 0,1 bis 34 Gew.-% mindestens eines anorganischen Salzes;
als Prozessgut in einer erfindungsgemäßen Vorrichtung zur Entspannungsverdampfung.

Gegenstand der vorliegenden Anmeldung ist zudem die Verwendung eines erfindungsgemäßen Produkts zur Beaufschlagung keratinhaltiger Fasern, insbesondere menschlicher Haare, mit einer kosmetischen Zubereitung a) bzw. zur temporären Verformung keratinhaltiger Fasern, insbesondere menschlicher Haare.

Ein Verfahren zur temporären Verformung keratinhaltiger Fasern, insbesondere menschlicher Haare, bei welchem die keratinischen Fasern mittels einer erfindungsgemäßen Vorrichtung zur Entspannungsverdampfung mit einer kosmetischen Zubereitung a) enthaltend bezogen auf ihr Gesamtgewicht
a1) 65 bis 96 Gew.-% mindestens eines polaren Lösungsmittels;
a2) 0,1 bis 34 Gew.-% mindestens eines anorganischen Salzes;
beaufschlagt werden, ist ein weiterer Gegenstand der vorliegenden Anmeldung. Die kosmetische Zubereitung a) wird mittels der Vorrichtung zur Entspannungsverdampfung vorzugsweise in einen Sprühnebel überführt, welcher nachfolgend die keratinhaltigen Fasern beaufschlagt.

Um eine ausreichende Sprühwirkung zu erzielen, wird die kosmetische Zubereitung a) dabei auf Temperaturen oberhalb des Siedepunktes des in der kosmetischen Zubereitung a) enthaltenen polaren Lösungsmittels oder Lösungsmittelgemisches erhitzt.

Handelt es sich bei dem polaren Lösungsmittel um Wasser oder Lösungsmittelgemische mit einem Wasseranteil oberhalb 50 Gew.-% (bezogen auf das Gesamtgewicht des Lösungsmittelgemisches), wird die kosmetische Zubereitung vorzugsweise auf Temperaturen oberhalb 100°C, vorzugsweise auf Temperaturen von 100°C und 240°C, besonders bevorzugt auf Temperaturen von 140°C bis 160°C erhitzt.

Der durch die Erhitzung der kosmetischen Zubereitung a) erzielte Überdruck beträgt in den Fällen, in denen es sich bei dem polaren Lösungsmittel um Wasser oder Lösungsmittelgemische mit einem Wasseranteil oberhalb 50 Gew.-% (bezogen auf das Gesamtgewicht des Lösungsmittelgemisches) handelt, vorzugsweise zwischen 1,1 und 8 bar, bevorzugt zwischen 1,2 und 4 bar.

Ein weiterer Anmeldungsgegenstand ist ein erfindungsgemäßes Verfahren zur temporären Verformung keratinhaltiger Fasern, insbesondere menschlicher Haare, bei welchem die keratinhaltigen Fasern mittels einer Vorrichtung zur Entspannungsverdampfung mit einer kosmetischen Zubereitung a) enthaltend bezogen auf ihr Gesamtgewicht
a1) 65 bis 96 Gew.-% mindestens eines polaren Lösungsmittels;
a2) 0,1 bis 34 Gew.-% mindestens eines anorganischen Salzes;
beaufschlagt werden, wobei
- aus einem Vorratsbehälter, in dessen Inneren ein Druck herrscht, der dem Umgebungsdruck entspricht, eine Teilmenge der in diesem Vorratsbehälter befindlichen kosmetischen Zubereitung a) in einen Behälter b1) überführt wird;
- nachfolgend der Zugang zwischen Vorratsbehälter und Behälter b1) durch ein Bauteil zur Durchflussregelung, mittels dessen der Durchfluss der kosmetischen Zubereitung a) aus dem Vorratsbehälter in den Behälter b1) unterbrochen werden kann, unterbrochen wird;
- nachfolgend die in dem gegen die Umgebung abgeschlossenen Behälter b1) befindliche kosmetische Zubereitung a) mittels einer Heizvorrichtung erhitzt wird, so dass der Druck im Inneren des Behälters b1) auf Werte oberhalb des Umgebungsdrucks, vorzugsweise auf Werte zwischen 1,1 und 8 bar, insbesondere auf Werte zwischen 1,2 und 4 bar ansteigt;
- nachfolgend der unter einem Druck oberhalb des Umgebungsdrucks stehende Behälter b1) in einer Weise geöffnet, wird, welche den Austritt mindestens einer Teilmenge, vorzugsweise mindestens 50 Gew.-%, bevorzugt mindestens 80 Gew.-% und insbesondere mindestens 90 Gew.-% der in dem Behälter b1) befindlichen kosmetischen Zubereitung aus dem Behälter b1) in die Umgebung unter Minderung des zum Zeitpunkt der Behälteröffnung in dem Behälter b1) herrschenden Drucks, entspannt wird.

Die Entspannung der kosmetischen Zubereitung a) in die Umgebung erfolgt vorzugsweise unter Ausbildung eines Sprühnebels der kosmetischen Zubereitung a).

Die aus dem Behälter b1) entspannte kosmetische Zubereitung a) wird vorzugsweise auf keratinische Fasern, insbesondere menschliche Haare aufgebracht.

Verfahren, in deren Verlauf die aus dem Behälter b1) entspannte kosmetische Zubereitung vor der Beaufschlagung der keratinischen Fasern durch eine Düse geleitet wird, sind besonders bevorzugt. Bezüglich weiterer bevorzugter Ausführungsformen der erfindungsgemäßen Verwendungen und des erfindungsgemäßen Verfahrens gilt mutatis mutandis das zu den erfindungsgemäßen kosmetischen Zubereitungen a) und zu der Vorrichtung zur Entspannungsverdampfung b) Gesagte.

## Patentansprüche

1. Kosmetisches Produkt, umfassend
a) eine flüssige kosmetische Zubereitung, enthaltend bezogen auf ihr Gesamtgewicht
a1) 65 bis 96 Gew.-% mindestens eines polaren Lösungsmittels;
a2) 0,1 bis 34 Gew.-% mindestens eines anorganischen Salzes;
b) eine Vorrichtung zur Entspannungsverdampfung der kosmetischen Zubereitung a) mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) unter Druckerhöhung auf eine Temperatur oberhalb des Siedepunktes des in der kosmetischen Zubereitung enthaltenen polaren Lösungsmittels zu erhitzen, sowie die erhitzte kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht.

2. Kosmetisches Produkt nach einem der vorherigen Ansprüche, **dadurch gekennzeichnet, dass** der Gewichtsanteil des polaren Lösungsmittels a1) am Gesamtgewicht der kosmetischen Zubereitung a) 75 bis 94 Gew.-%, vorzugsweise 85 bis 92 Gew.-% beträgt.

3. Kosmetisches Produkt nach einem der vorherigen Ansprüche, **dadurch gekennzeichnet, dass** der Gewichtsanteil des anorganischen Salzes a2) am Gesamtgewicht der kosmetischen Zubereitung a) 1,0 bis 24 Gew.-%, vorzugsweise 2,0 bis 14 Gew.-% beträgt.

4. Kosmetisches Produkt nach einem der vorherigen Ansprüche, **dadurch gekennzeichnet, dass** das anorganische Salz a2) ausgewählt ist aus der Gruppe der Chloride und Sulfate der Alkali- und Erdalkalimetalle, insbesondere aus der Gruppe Magnesiumsulfat, Magnesiumchlorid und Natriumchlorid.

5. Kosmetisches Produkt nach einem der vorherigen Ansprüche, **dadurch gekennzeichnet, dass** die kosmetische Zubereitung a) bezogen auf ihr Gesamtgewicht 0,05 bis 4,0 Gew.-%, vorzugsweise 0,1 bis 2,0 Gew.-% und insbesondere 0,2 bis 1,0 Gew.-% nichtionisches Tensid a3) enthält.

6. Kosmetisches Produkt nach einem der vorherigen Ansprüche, **dadurch gekennzeichnet, dass** die kosmetische Zubereitung a) bezogen auf ihr Gesamtgewicht 0,1 bis 10 Gew.-%, vorzugsweise 0,2 bis 8,0 Gew.-%, und insbesondere 0,5 bis 5,0 Gew.-% eines Polyalkylenglycols a4) enthält.

7. Verwendung einer flüssigen kosmetischen Zubereitung a) enthaltend, bezogen auf ihr Gesamtgewicht,
a1) 65 bis 96 Gew.-% mindestens eines polaren Lösungsmittels;
a2) 0,1 bis 34 Gew.-% mindestens eines anorganischen Salzes;
als Prozessgut in einer Vorrichtung zur Entspannungsverdampfung der kosmetischen Zubereitung a) mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) unter Druckerhöhung auf eine Temperatur oberhalb des Siedepunktes des in der kosmetischen Zubereitung enthaltenen polaren Lösungsmittels zu erhitzen, sowie die erhitzte kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht.

8. Verwendung eines Produkts nach einem der Ansprüche 1 bis 6 zur Beaufschlagung keratinhaltiger Fasern, insbesondere menschlicher Haare, mit einer kosmetischen Zubereitung a).

9. Verwendung eines Produkts nach einem der Ansprüche 1 bis 6 zur temporären Verformung keratinhaltiger Fasern, insbesondere menschlicher Haare.

10. Verfahren zur temporären Verformung keratinhaltiger Fasern, insbesondere menschlicher Haare, bei welchem die keratinhaltigen Fasern mittels einer Vorrichtung zur Entspannungsverdampfung einer flüssigen kosmetischen Zubereitung mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung a) befüllten Innenraum des Behälters zu verschließen und zu öffnen,
b3) einer Heizvorrichtung, um die in dem geschlossenen Innenraum des Behälters befindliche kosmetische Zubereitung a) unter Druckerhöhung auf eine Temperatur oberhalb des Siedepunktes des in der kosmetischen Zubereitung enthaltenen polaren Lösungsmittels zu erhitzen, sowie die erhitzte kosmetische Zubereitung a) aus dem Innenraum des Behälters unter Druckminderung in die Umgebung zu entspannen,
b4) einer Düse, welche eine Zerstäubung der aus dem Behälter entweichenden kosmetischen Zubereitung a) ermöglicht,
mit einer kosmetischen Zubereitung a) enthaltend bezogen auf ihr Gesamtgewicht
a1) 65 bis 96 Gew.-% mindestens eines polaren Lösungsmittels;
a2) 0,1 bis 34 Gew.-% mindestens eines anorganischen Salzes; beaufschlagt werden.

11. Verfahren nach Anspruch 10, **dadurch gekennzeichnet, dass**
- aus einem Vorratsbehälter, in dessen Inneren ein Druck herrscht, der dem Umgebungsdruck entspricht, eine Teilmenge der in diesem Vorratsbehälter befindlichen kosmetischen Zubereitung a) in einen Behälter überführt wird;
- nachfolgend der Zugang zwischen Vorratsbehälter und Behälter durch ein Bauteil zur Durchflussregelung, mittels dessen der Durchfluss der kosmetischen Zubereitung a) aus dem Vorratsbehälter in den Behälter unterbrochen werden kann, unterbrochen wird;
- nachfolgend die in dem gegen die Umgebung abgeschlossenen Behälter befindliche kosmetische Zubereitung a) mittels einer Heizvorrichtung erhitzt wird, so dass der Druck im Inneren des Behälters auf Werte oberhalb des Umgebungsdrucks, vorzugsweise auf Werte zwischen 1,1 und 8 bar, insbesondere auf Werte zwischen 1,2 und 4 bar ansteigt;
- nachfolgend der unter einem Druck oberhalb des Umgebungsdrucks stehende Behälter in einer Weise geöffnet, wird, welche den Austritt mindestens einer Teilmenge, vorzugsweise mindestens 50 Gew.-%, bevorzugt mindestens 80 Gew.-% und insbesondere mindestens 90 Gew.-% der in dem Behälter befindlichen kosmetischen Zubereitung aus dem Behälter in die Umgebung unter Minderung des zum Zeitpunkt der Behälteröffnung in dem Behälter herrschenden Drucks, entspannt wird.

## Claims

1. A cosmetic product, comprising
a) a liquid cosmetic preparation containing, based on the total weight thereof,
a1) 65 to 96 wt.% of at least one polar solvent;
a2) 0.1 to 34 wt.% of at least one inorganic salt;
b) a device for flash evaporation of the cosmetic preparation a), comprising
b1) a container defining a closed interior in which the cosmetic preparation can be received,
b2) a valve or a similarly functioning closure element for closing and opening the interior of the container which is at least partly filled with the cosmetic preparation a),
b3) a heating device for heating, under increased pressure, the cosmetic preparation a) which is located in the closed interior of the container to a temperature above the boiling point of the polar solvent contained in the cosmetic preparation, and for releasing, under reduced pressure, the heated cosmetic preparation a) from the interior of the container into the surroundings,
b4) a nozzle which allows atomization of the cosmetic preparation a) escaping from the container.

2. The cosmetic product according to one of the preceding claims, **characterized in that** the weight proportion of the polar solvent a1) with respect to the total weight of the cosmetic preparation a) is 75 to 94 wt.%, preferably 85 to 92 wt.%.

3. The cosmetic product according to one of the preceding claims, **characterized in that** the weight proportion of the inorganic salt a2) with respect to the total weight of the cosmetic preparation a) is 1.0 to 24 wt.%, preferably 2.0 to 14 wt.%.

4. The cosmetic product according to one of the preceding claims, **characterized in that** the inorganic salt a2) is selected from the group of chlorides and sulfates of alkali and alkaline earth metals, in particular from the group of magnesium sulfate, magnesium chloride and sodium chloride.

5. The cosmetic product according to one of the preceding claims, **characterized in that** the cosmetic preparation a) contains, based on the total weight thereof, 0.05 to 4.0 wt.%, preferably 0.1 to 2.0 wt.%, and in particular 0.2 to 1.0 wt.%, non-ionic surfactant a3).

6. The cosmetic product according to one of the preceding claims, **characterized in that** the cosmetic preparation a) contains, based on the total weight thereof, 0.1 to 10 wt.%, preferably 0.2 to 8.0 wt.%, and in particular 0.5 to 5.0 wt.%, of a polyalkylene glycol a4).

7. The use of a liquid cosmetic preparation a) containing, based on the total weight thereof,
a1) 65 to 96 wt.% of at least one polar solvent;
a2) 0.1 to 34 wt.% of at least one inorganic salt;
as a process material in a device for flash evaporation of the cosmetic preparation a), comprising
b1) a container defining a closed interior in which the cosmetic preparation can be received,
b2) a valve or a similarly functioning closure element for closing and opening the interior of the container which is at least partly filled with the cosmetic preparation a),
b3) a heating device for heating, under increased pressure, the cosmetic preparation a) which is located in the closed interior of the container to a temperature above the boiling point of the polar solvent contained in the cosmetic preparation, and for releasing, under reduced pressure, the heated cosmetic preparation a) from the interior of the container into the surroundings,
b4) a nozzle which allows atomization of the cosmetic preparation a) escaping from the container.

8. The use of a product according to one of claims 1 to 6 for applying a cosmetic preparation a) to keratin-containing fibers, in particular human hair.

9. The use of a product according to one of claims 1 to 6 for temporarily shaping keratin-containing fibers, in particular human hair.

10. A method for temporarily shaping keratin-containing fibers, in particular human hair, in which method, by means of a device for flash evaporation of a liquid cosmetic preparation, said device comprising
b1) a container defining a closed interior in which the cosmetic preparation can be received,
b2) a valve or a similarly functioning closure element for closing and opening the interior of the container which is at least partly filled with the cosmetic preparation a),
b3) a heating device for heating, under increased pressure, the cosmetic preparation a) which is located in the closed interior of the container to a temperature above the boiling point of the polar solvent contained in the cosmetic preparation, and for releasing, under reduced pressure, the heated cosmetic preparation a) from the interior of the container into the surroundings,
b4) a nozzle which allows atomization of the cosmetic preparation a) escaping from the container,
a cosmetic preparation a), containing, based on the total weight thereof,
a1) 65 to 96 wt.% of at least one polar solvent;
a2) 0.1 to 34 wt.% of at least one inorganic salt;
is applied to the keratin-containing fibers.

11. The method according to claim 10, **characterized in that**
- some of the cosmetic preparation a) located in a reservoir, in the interior of which a pressure prevails that corresponds to the ambient pressure, is transferred from said reservoir into a container;
- using a component for controlling flow, by means of which the flow of the cosmetic preparation a) from the reservoir into the container can be interrupted, access between the reservoir and the container is subsequently interrupted;
- the cosmetic preparation a) located in the container, which is sealed against the surroundings, is subsequently heated by means of a heating device such that the pressure inside the container increases to values above the ambient pressure, preferably to values between 1.1 and 8 bar, in particular to values between 1.2 and 4 bar;
- the container, which is pressurized above the ambient pressure, is subsequently opened in a manner in which the discharge of at least some, preferably at least 50 wt.%, more preferably at least 80 wt.%, and in particular at least 90 wt.%, of the cosmetic preparation located in the container is released from the container into the surroundings by the pressure that prevails in the container at the time at which the container is opened being reduced.

## Revendications

1. Produit cosmétique, comprenant
a) une préparation cosmétique liquide contenant, par rapport à son poids total,
a1) de 65 à 96 % en poids d'au moins un solvant polaire ;
a2) de 0,1 à 34 % en poids d'au moins un sel inorganique ;
b) un dispositif de vaporisation par détente de la préparation cosmétique a) comportant
b1) un récipient définissant un espace intérieur fermé dans lequel la préparation cosmétique peut être reçue,
b2) une soupape ou un élément de fermeture à action comparable afin de fermer et d'ouvrir l'espace intérieur du récipient rempli au moins partiellement avec la préparation cosmétique a),
b3) un dispositif de chauffage afin de chauffer la préparation cosmétique a) présente dans l'espace intérieur fermé du récipient tout en augmentant la pression à une température supérieure au point d'ébullition du solvant polaire contenu dans la préparation cosmétique, et de détendre la préparation cosmétique a) chauffée hors de l'espace intérieur du récipient tout en réduisant la pression dans l'environnement,
b4) une buse permettant d'atomiser la préparation cosmétique a) s'échappant du récipient.

2. Produit cosmétique selon l'une des revendications précédentes, **caractérisé en ce que** le pourcentage en poids du solvant polaire a1) dans le poids total de la préparation cosmétique a) se situe dans la plage allant de 75 à 94 % en poids, de préférence de 85 à 92 % en poids.

3. Produit cosmétique selon l'une des revendications précédentes, **caractérisé en ce que** le pourcentage en poids du sel inorganique a2) dans le poids total de la préparation cosmétique a) se situe dans la plage allant de 1,0 à 24 % en poids, de préférence de 2,0 à 14 % en poids.

4. Produit cosmétique selon l'une des revendications précédentes, **caractérisé en ce que** le sel inorganique a2) est choisi dans le groupe des chlorures et des sulfates de métaux alcalins et alcalino-terreux, en particulier dans le groupe du sulfate de magnésium, du chlorure de magnésium et du chlorure de sodium.

5. Produit cosmétique selon l'une des revendications précédentes, **caractérisé en ce que** la préparation cosmétique a) contient, par rapport à son poids total, 0,05 à 4,0 % en poids, de préférence 0,1 à 2,0 % en poids et en particulier 0,2 à 1,0 % en poids d'un tensioactif non ionique a3).

6. Produit cosmétique selon l'une des revendications précédentes, **caractérisé en ce que** la préparation cosmétique a) contient, par rapport à son poids total, 0,1 à 10 % en poids, de préférence 0,2 à 8,0 % en poids, et en particulier 0,5 à 5,0 % en poids d'un polyalkylène glycol a4).

7. Utilisation d'une préparation cosmétique a) liquide contenant, par rapport à son poids total,
a1) de 65 à 96 % en poids d'au moins un solvant polaire ;
a2) de 0,1 à 34 % en poids d'au moins un sel inorganique ;
en tant que produit de traitement dans un dispositif de vaporisation par détente de la préparation cosmétique a) comportant
b1) un récipient définissant un espace intérieur fermé dans lequel la préparation cosmétique peut être reçue,
b2) une soupape ou un élément de fermeture à action comparable afin de fermer et d'ouvrir l'espace intérieur du récipient rempli au moins partiellement avec la préparation cosmétique a),
b3) un dispositif de chauffage afin de chauffer la préparation cosmétique a) présente dans l'espace intérieur fermé du récipient tout en augmentant la pression à une température supérieure au point d'ébullition du solvant polaire contenu dans la préparation cosmétique, et de détendre la préparation cosmétique a) chauffée hors de l'espace intérieur du récipient tout en réduisant la pression dans l'environnement,
b4) une buse permettant d'atomiser la préparation cosmétique a) s'échappant du récipient.

8. Utilisation d'un produit selon l'une des revendications 1 à 6 pour l'application d'une préparation cosmétique a) sur des fibres kératiniques, en particulier sur des cheveux humains.

9. Utilisation d'un produit selon l'une des revendications 1 à 6 pour la mise en forme temporaire de fibres kératiniques, en particulier de cheveux humains.

10. Procédé de mise en forme temporaire de fibres kératiniques, en particulier de cheveux humains, selon lequel on applique sur les fibres kératiniques, au moyen d'un dispositif d'évaporation par détente d'une préparation cosmétique comportant
b1) un récipient définissant un espace intérieur fermé dans lequel la préparation cosmétique peut être reçue,
b2) une soupape ou un élément de fermeture à action comparable afin de fermer et d'ouvrir l'espace intérieur du récipient rempli au moins partiellement avec la préparation cosmétique a),
b3) un dispositif de chauffage afin de chauffer la préparation cosmétique a) présente dans l'espace intérieur fermé du récipient tout en augmentant la pression à une température supérieure au point d'ébullition du solvant polaire contenu dans la préparation cosmétique, et de détendre la préparation cosmétique a) chauffée hors de l'espace intérieur du récipient tout en réduisant la pression dans l'environnement,
b4) une buse permettant de vaporiser la préparation cosmétique a) s'échappant du récipient, une préparation cosmétique a) contenant, par rapport à son poids total,
a1) de 65 à 96 % en poids d'au moins un solvant polaire ;
a2) de 0,1 à 34 % en poids d'au moins un sel inorganique.

11. Procédé selon la revendication 10, **caractérisé en ce que**,
- à partir d'un réservoir de stockage à l'intérieur duquel règne une pression égale à la pression ambiante, une fraction de la préparation cosmétique a) présente dans ce réservoir de stockage est transférée dans un récipient ;
- l'accès entre le réservoir de stockage et le récipient est ensuite interrompu par un élément de régulation d'écoulement au moyen duquel l'écoulement de la préparation cosmétique a) du réservoir de stockage au récipient peut être interrompu ;
- la préparation cosmétique a) présente dans le récipient isolé de l'environnement est ensuite chauffée au moyen d'un dispositif de chauffage, de sorte que la pression à l'intérieur du récipient dépasse la pression ambiante et atteint de préférence des valeurs comprises entre 1,1 et 8 bar, en particulier entre 1,2 et 4 bar ;
- le récipient se trouvant à une pression supérieure à la pression ambiante est ensuite ouvert de manière à détendre au moins une fraction, de préférence au moins 50 % en poids, préférentiellement au moins 80 % en poids et en particulier au moins 90 % en poids de la préparation cosmétique présente dans le récipient et à provoquer sa sortie dans l'environnement tout en réduisant la pression régnant dans le récipient au moment de l'ouverture de celui-ci.
